# FASCICULE DE BREVET EUROPEEN

(11) **EP 1 156 839 B1**
(45) Date de publication et mention de la délivrance du brevet: **26.10.2005**
(21) Numéro de dépôt: 00909403.8
(22) Date de dépôt: 01.03.2000
(51) Int. Cl.: A61L 31/06

(54) **PEPTIDE COLLAGENIQUE RETICULE POUR LA PREVENTION DES ADHERENCES POST-CHIRURGICALES**
PEPTID AUF KOLLAGENBASIS ZUR VERHINDERUNG VON POSTOPERATIVEN VERKLEBUNGEN
CROSS-LINKED COLLAGEN PEPTIDE FOR PREVENTING POST-SURGICAL ADHESIONS

(30) Priorité: 02.03.1999 FR 9902728
(43) Date de publication de la demande: 28.11.2001
(73) Titulaire: FLAMEL TECHNOLOGIES, 69603 Vénissieux (FR)
(72) Inventeur: CONSTANCIS, Alain, F-69008 Lyon (FR); MEYRUEIX, Rémi, F-69009 Lyon (FR)
(74) Mandataire: Fleurance, Raphael
(86) Numéro de dépôt international: PCT/FR2000/000514
(87) Numéro de publication internationale: WO 2000/051661

(56) Documents cités:
- US-A- 5 412 076
- US-A- 5 786 421
- NICOLAS F L ET AL: "Denatured thiolated collagen. I. Synthesis and characterization" BIOMATERIALS, GB, ELSEVIER SCIENCE PUBLISHERS BV., BARKING, vol. 18, no. 11, page 807-813 XP004063906 ISSN: 0142-9612
- NICOLAS F L ET AL: "Denatured thiolated collagen. II. Cross-linking by oxidation" BIOMATERIALS, GB, ELSEVIER SCIENCE PUBLISHERS BV., BARKING, vol. 18, no. 11, page 815-821 XP004063907 ISSN: 0142-9612

## Description

Le domaine de l'invention est celui des biomatériaux médicaux ou chirurgicaux, bio-compatibles, bio-résorbables, utiles dans la prévention des adhésions post-opératoires, plus particulièrement à l'issue des interventions chirurgicales intrapéritonéales et pelviennes.

L'invention concerne un moyen, plus spécialement mais non limitativement un film, un gel ou un liquide (applicable e. g. par pulvérisation) pour la prévention de ces adhérences post-chirurgicales, constitué d'un peptide collagénique spécifique, chimiquement modifié, réticulé *in situ* ou mis en oeuvre à l'état réticulé.

L'invention vise également un procédé d'obtention, parmi d'autres, du moyen susmentionné.

L'un des problèmes majeurs rencontré en chirurgie, notamment digestive abdominale et gynécologique, est lié à la formation d'adhérences post-chirurgicales (accolement anormal entre deux surfaces ou segments de tissus normalement séparés) qui résulte d'une activité inflammatoire et/ou cicatricielle automatiquement générée suite à un traumatisme des tissus engendré par l'intervention. De telles adhésions délocalisées peuvent avoir des répercussions fâcheuses. Ainsi, en chirurgie abdominale, ces adhésions peuvent entrainer des obstructions. En chirurgie gynécologique, les adhésions pelviennes post-opératoires sont l'une des causes reconnues du manque d'efficacité du traitement chirurgical de la stérilité.

Pour tenter de remédier à ce problème, des approches chimiques et/ou thérapeutiques ont été proposées.
L'approche chimique consiste à appliquer sur les plaies chirurgicales des agents de traitement chimique, aptes à réfréner les phénomènes physiologiques d'inflammation et de cicatrisation à l'origine des adhésions. Ces agents de traitement sont des enzymes tels que la fibrinolysine et la papase ou bien encore des produits tels que la phénylbutazone, la prédnisolone, la polyvinylpyrrolidone ou les dextranes.
L'approche physique consiste à isoler la plaie chirurgicale du tissu avoisinant en intercalant une barrière physique, qui peut être un film ou un pansement non fibreux ou fibreux, tissé ou atissé ou sous forme de gel. Cette barrière physique empêche l'adhésion au cours de la cicatrisation. Mais dès lors que cette dernière n'a plus cours et donc que les risques d'adhésion sont inexistants, cette barrière physique ne représente rien d'autre qu'un corps étranger inutile, voire même gênant ,pour ne pas dire dangereux dans certains cas.
D'où il s'ensuit que les moyens anti-adhésifs connus en matériaux polymère du type polytétrafluoroéthylène ou silicone qui, bien qu'ayant fait leurs preuves en matière d'anti-adhésion, n'en demeurent pas moins non biodégradables et doivent donc être retirés lors d'une seconde opération chirurgicale. Cette pratique doublement traumatisante pour le patient a pour autre inconvénient de déplacer le problème de l'adhésion post-chirurgicale d'une région tissulaire vers une autre lésée lors de la deuxième opération (laparotomie).

Ainsi, pour ne pas en rester là, on a essayé de développer des moyens de prévention des adhérences chirurgicales post-opératoires, constitués par des matériaux bio-dégradables (ou bio-résorbables), au terme de la cicatrisation, dès lors que leur fonction de barrière a été remplie et qu'aucun risque d'adhésion ne persiste.
Connaissant les présumées propriétés de biodégradabilité et biocompatibilité des polymères naturels et de leurs dérivés, certains chercheurs ont proposé de mettre en oeuvre des peptides collagéniques (collagène, gélatine), des polysaccharides (cellulose-amidon et dérivés), des mucopolysaccharides, entre autres, comme matériau constitutif d'implants ou des prothèses chirurgicales et notamment de barrières anti-adhésions post chirurgicales.

La Société Johnson & Johnson Products inc,. a mis au point un matériau bio-résorbable à base de cellulose régénérée, oxydée, utile comme barrière anti-adhésion post-opératoire et se présentant sous forme de tissu ayant une densité de 8 à 15 mg/cm² et une porosité de 10 à 20 %. (EP-A-0 213 563). La Société JOHNSON & JOHNSON a également développé un procédé pour la préparation de cellulose oxydée neutralisée dont l'intégrité et la résistance à la traction sont préservées, en vue d'utilisations, notamment, comme moyen pour la prévention des adhérences post-chirurgicales (EP-A-0 437 095). En prolongement à cela, cette Société a également mis au point un film multicouches en cellulose oxydée et en cellulose, utile comme barrière anti-adhésion ou comme pansement (EP-A-0 815 881).

Dans les brevets US-A-5 017 229 et US-A-5 527 893, les inventeurs BURNS et al. de la Société GENZYME CORPORATION décrivent une barrière pour la prévention des adhérences post-chirurgicales constituée par un polysaccharide polyanionique, à savoir la carboxyméthylcellulose, associé à de l'acide hyaluronique, de manière à former un gel.

De manière générale, les applications des matériaux collagéniques comme films chirurgicaux sont connus depuis longtemps. Ainsi, le brevet anglais GB-A-1 095 552 de 1964 décrit un film de collagène formé par une multiplicité de filaments non tissés.

Le brevet français FR-A-2 628 634 décrit un patch de chirurgie viscérale réalisé à partir d'un biomatériau formé de deux couches de collagène superposées et associées intimement, à savoir une couche poreuse collable de collagène fibreux et un film de collagène et/ou de gélatine. Ce patch vise à permettre une bonne cicatrisation des viscères. Il s'agit d'un pansement destiné à remplacer temporairement la paroi viscérale à reconstituer. Il est présenté comme étant entièrement biodégradable et comme étant apte à réaliser un excellent effet de confinement et d'hémostase. Il ne s'agit pas là spécifiquement d'une application de prévention des adhérences post-chirurgicales.

La demande de brevet PCT WO 96/08 277 concerne l'utilisation de membranes collagéniques comme prothèse de régénération péritonéale. Le matériau collagénique considéré est un gel de collagène éventuellement réticulé, transparent, bio-compatible, suturable ou agrafage et bio-résorbable. Le collagène mis en oeuvre est un collagène avec ou sans telopeptides, éventuellement associé à un glycosoaminoglycanne. Il est à noter que l'agent de réticulation éventuellement mis en oeuvre est un composé chimique réactif (glutaraldéhyde, diphénylphosphorylazide, carbodiimide) qui n'est pas neutre en terme de toxicité.

Dans le brevet FR-A-2 628 634, le patch est formé par deux couches de collagène superposées et associées intimement. La première couche est formée par du collagène fibreux ce qui lui confère une nature poreuse et collable. La deuxième couche est obtenue par collage d'un film de collagène ou de gélatine sur la première couche de collagène fibreux. Ce patch présente, selon les inventeurs du brevet PCT WO 96/08277, une résistance mécanique relativement faible. Le document FR-A-2 628 634 est silencieux vis-à-vis de la prévention des adhérences post-chirurgicales.

La demande de brevet européen EP-A-0 686 402 divulgue une membrane collagénique pour la prévention des adhérences post-chirurgicales, comprenant un support à base de collagène revêtu d'une couche de gélatine non mélangée au support. Cette membrane se présente, de préférence, sous forme lyophilisée. Le collagène mis en oeuvre peut être réticulé à l'aide de diphénylphosphorylazyde (DPPA).Là encore le choix de ce genre de collagène réticulé n'est pas de bon aloi en raison des problèmes de toxicité qu'il sous-tend.

La demande de brevet français FR-A-2 759 083 décrit un matériau collagénique utile notamment pour la prévention d'adhérences post-opératoires. Ce matériau collagénique serait d'après les inventeurs, bio-compatible, non toxique, potentiellement adhésif et biodégradable en moins d'une semaine. Ce matériau collagénique comprend du collagène modifié par coupure oxydative et chauffage au-dessus de 37 °C et réticulé en présence d'au moins un additif hydrophile macromoléculaire chimiquement non réactif vis-à-vis du collagène. L'agent hydrophile est, par exemple, du polyéthylèneglycol ou un polysaccharide tel que l'amidon, le dextrane ou la cellulose.

Il ressort de cette revue de l'art antérieur qu'aucun des matériaux biodégradables, notamment cellulosiques ou collagéniques, utilisés jusqu'alors dans la prévention des adhérences post-chirurgicales, ne donne satisfaction, notamment pour les raisons énoncées ci-après.
- Certains peuvent avoir une toxicité résiduelle, compte tenu de leur mode de réticulation chimique, par exemple à l'aide d'aldéhydes.
- La biodégradabilité n'est pas suffisamment contrôlable pour pouvoir être mise en adéquation avec le maintien de leur fonction de barrière physique entre les tissus, pendant et uniquement pendant la durée de cicatrisation au cours de laquelle les risques d'adhérence chirurgicale existent.
- Certains ont une résistance mécanique initiale très faible.
- S'agissant des matériaux collagéniques, ils ne peuvent être mis sous forme de solutions filtrables sur des filtres dont la porosité est suffisamment faible pour permettre la rétention de contaminants biologiques (stérilisation).

L'un des objectifs essentiels de la présente invention est de fournir un moyen, à base de collagène, pour la prévention des adhérences post-chirurgicales, ce moyen se devant de satisfaire à un cahier des charges remédiant aux inconvénients susvisés des matériaux de l'art antérieur.

Un autre objectif essentiel de l'invention est de fournir un moyen pour la prévention des adhérences post-chirurgicales, comprenant un matériau collagénique et apte à pouvoir servir de support pour un ou plusieurs agents chimiques de lutte contre les adhérences post-chirurgicales.

Un autre objectif essentiel de l'invention est de fournir un moyen pour la prévention des adhérences post-chirurgicales, comprenant un collagène modifié et réticulé pouvant être fabriqué sous forme de film ou de membrane. Ce film ou membrane peut être constitué d'un matériau composite comprenant , d'une part, du collagène modifié chimiquement et réticulé satisfaisant aux exigences susvisées et, d'autre part, un matériau de renfort, tissé ou non, de préférence biodégrabable, de nature collagénique ou non collagénique.

Un autre objectif essentiel de l'invention est de fournir un moyen pour la prévention des adhérences post-chirurgicales constitué par une forme liquide (e. g. : liquide pulvérisable ou gel) de viscosité adaptée comprenant du collagène modifié, au moins en partie non réticulé, réticulable in situ sur les tissus biologiques.

Un autre objectif essentiel de l'invention est de fournir un moyen pour la prévention des adhérences post-chirurgicales qui soit économique, facile à obtenir, à manipuler et à mettre en oeuvre.

S'étant fixés tous ces objectifs, les inventeurs ont mené de longues et laborieuses recherches et expérimentations au terme desquelles ils ont pu mettre au point de manière tout à fait surprenante et inattendue un nouveau peptide collagénique, modifié chimiquement, réticulable et/ou réticulé par l'intermédiaire de ponts disulfure reliés aux motifs carboxyliques des acides aspartique et glutamique des chaînes collagéniques par des liaisons amides. Ce nouveau collagène réticulable/réticulé s'est avéré particulièrement approprié comme élément constitutif essentiel, d'un moyen - de facto, nouveau lui aussi - pour la prévention des adhérences post-chirurgicales.

D'où il s'ensuit que la présente invention satisfait aux objectifs visés ci-dessus, parmi d'autres, en fournissant un moyen pour la prévention des adhérences post-chirurgicales, caractérisé en ce qu'il comprend au moins un peptide collagénique modifié par greffage de fonctions thiol libres ou substituées, réticulable et/ou au moins en partie réticulé et dont les fonctions thiol sont apportées par des restes mercaptoaminés exclusivement greffés sur les acides aspartique et glutamique des chaînes collagéniques, par l'intermédiaire de liaisons amides.

Au sens de la présente invention, le terme "peptide collagénique" désigne notamment le collagène avec (natif) ou sans télopeptide, le collagène dénaturé ainsi que la gélatine.

Le brevet US 5,412,076 décrit également du collagène réticulable modifié par greffage de fonctions thiol appartenant à des restes mercaptoaminés fixés sur le collagène par l'intermédiaire d'un espaceur du type acide dicarboxylique. Les fonctions carboxyliques de ce dernier réagissents avec les groupements alcool ou amine libres du collagène. Le reste mercaptoaminé, quant à lui, réagit par l'intermédiaire de sa fonction amine libre avec les groupements carboxyliques présents sur la chaîne collagénique. Ces groupements sont ainsi de deux types : ceux apportés par l'espaceur et ceux existant à l'état naturel dans le collagène, c'est-à-dire portés par les acides aspartiques et glutamiques.

Contrairement au brevet US 5,412,076, le peptide collagénique sélectionné conformément à l'invention, a ceci de particulier que les fonctionnalités de réticulation sont portées exclusivement par des restes carboxyle des acides aspartique et glutamique de la chaîne collagénique .Cela lui confère des propriétés avantageuses inattendues sur le plan de la mécanique, de la solubilité en milieu aqueux, de l'adhérence et de la biologie (biodégradation).
Ce peptide collagénique modifié chimiquement peut exister sous au moins trois formes différentes :
**A**. précurseur à fonctions thiols substituées,
**B**. précurseur réticulable à fonctions thiols libres,
**C**. forme réticulée -S-S-.
La forme **B** peut être obtenue à partir de **A** et la forme **C** à partir de la forme **B.**
Le moyen de prévention des adhérences selon l'invention comprend au moins l'une des formes **A, B** ou **C**. Il est clair que les formes **A** et **B** correspondent plus spécialement au moyen à l'état de conditionnement et de stockage du moyen avant application sur le site où il est destiné à exprimer sa fonction d'antiadhérence. Et c'est sous forme réticulée **C** que ledit moyen est le plus à même d'exprimer cette fonction.
Sous forme réticulée **C**, le peptide collagénique, modifié chimiquement, entrant dans la composition du moyen selon l'invention, peut avoir de fortes densités de pontages disulfure, lui procurant une excellente stabilité ainsi que de bonnes propriétés élastiques et une grande résistance mécanique. Un autre avantage de ce peptide collagénique judicieusement sélectionné, tient au fait que son niveau de réticulation peut être contrôlable. Enfin, la modification chimique par greffage de restes mercaptoaminés du peptide collagénique considéré ne porte pas ou peu préjudice à sa bio-compatibilité.

L'utilisation de ce peptide collagénique au moins en partie réticulé (**C**) comme moyen pour la prévention des adhérences post-chirurgicales, est tout à fait judicieuse et originale. Elle permet de disposer d'un nouveau moyen d'anti-adhésion faisant barrière entre les tissus, de manière tout à fait efficace et en étant parfaitement bien toléré par l'organisme des patients. Sa bio-résorbabilité peut être réglée de telle sorte que le moyen conserve sa fonction d'effet barrière pendant le temps nécessaire à la cicatrisation.

Le moyen selon l'invention peut donner naissance à différents produits manufacturés du type films, gels, liquides pulvérisables, pansements, gazes, compresses, emplâtres, etc.

Une autre disposition avantageuse de ce moyen, résulte du fait que les formes **A** et **B** intermédiaires peuvent se présenter à l'état de solution filtrable sur des mailles inférieures à 0,22 microns. Cela permet d'effectuer rapidement et aisément une stérilisation, incontournable pour des produits destinés à être implantés in-vivo.

Avantageusement, au moins une partie du peptide collagénique modifié constitutif du moyen selon l'invention est sous forme d'un précurseur **A** sur lequel sont greffés des restes mercaptoaminés porteurs de fonctions thiols substituées, au moins une partie de ces restes mercaptoaminés répondant à la formule générale (I) suivante : 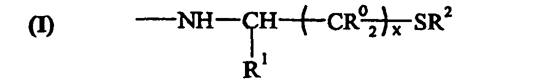 dans laquelle :
- x = 1 ou 2,
- R⁰ = H ou CH₃,
- R¹ représente H ou COOR³ avec R³ correspondant à un radical hydrocarboné de type aliphatique, aromatique ou alicyclique, de préférence alkylique, alcénylique, arylique, aralkylique, alkylarylique, aralcénylique, alcénylarylique et, plus préférentiellement encore, de type méthylique ou éthylique ;
- R² est un radical aliphatique et/ou alicylique et/ou aromatique, de préférence un alkyle ou un acyle éventuellement soufré et/ou aminé, et plus préférentiellement encore R² répond à la formule (**II**) suivante : 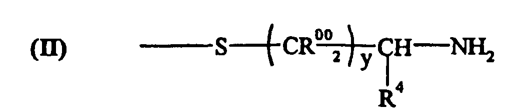
- avec y, R⁰⁰ et R⁴ répondant à la même définition que celle donnée en légende dans la formule (I) pour x, R⁰ et R¹.

En pratique, les restes mercaptoaminés, greffés sur la forme A du peptide collagénique, sont choisis dans le groupe des radicaux suivants : 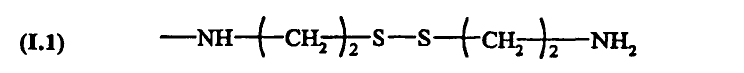 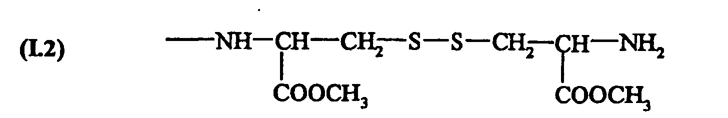 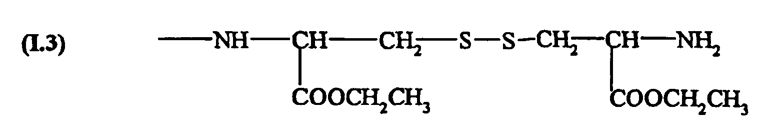 Pour transformer le précurseur **A** dans lequel **R**^{**2**} correspond à : -S-*radical hydrocarboné* (thiols substitués), en précurseur réticulable **B** intermédiaire de type thiol, on procède à une réduction à l'aide d'agents réducteurs connus tels que des mercaptans (mercaptoéthanol, acide mercaptoacétique, mercaptoéthylamine, benzylmercaptan, thiocrésol, dithiothréitol...) et/ou des sels réducteurs (NaBH₄, Na₂SO₃...) et/ou des réducteurs organiques (phosphine).
Ainsi, suivant une caractéristique préférée de l'invention, au moins une partie du peptide collagénique modifié est sous une forme **B** de précurseur réticulable intermédiaire de type thiol, sur lequel sont greffés des restes mercaptoaminés dont au moins une partie répond à la formule générale **(I)** donnée ci-dessus et dans laquelle le substituant R² correspond à l'hydrogène et dans laquelle **R**^{**3**} peut représenter l'hydrogène ou un sel (Na⁺, K⁺, Li⁺), outre les groupements hydrocarbonés, tels que définis supra en légende de la formule **(I),** pour autant que l'on déprotège l'ester.

S'agissant des précurseurs réticulables **B** de type thiol correspondant à la formule (**I**) dans laquelle R² = H, leur transformation en peptide collagénique réticulé **C** (réticulation), s'opère par oxydation des thiols en ponts disulfures. Cela permet d'obtenir un réseau collagénique tridimensionnel, insoluble dans les milieux physiologiques et solubles dans les milieux réducteurs capables de réduire les ponts disulfure. Cette oxydation peut intervenir spontanément en présence de l'oxygène de l'air, avantageusement en milieu faiblement basique, et éventuellement en présence d'agents auxiliaires oxydants tels que l'eau oxygénée ou les dérivés iodés (solution d'iode, bétadine).

Ainsi, suivant une modalité préférée du moyen selon l'invention, au moins une partie du peptide collagénique modifié est une forme **C** réticulée comprenant des chaînes collagéniques reliées entre elles par des ponts disulfure dont les atomes de soufre constitutifs appartiennent à des restes mercaptoaminés exclusivement greffés, sur les acides aspartique et glutamique des chaînes collagéniques, par l'intermédiaire de liaisons amides.

De manière plus préférée encore, le peptide collagénique sous forme **C** est obtenu à partir du peptide collagénique **B**.

Ces peptides collagéniques réticulés **C** ont un niveau de réticulation contrôlable en jouant sur le taux de substitution des motifs carboxyliques des résidus acides aspartique et glutamique des chaînes collagéniques. On dispose ainsi d'une certaine marge de manoeuvre pour choisir la qualité mécanique des matériaux appropriés à l'application visée.
Les ponts disulfure de ces peptides collagéniques réticulés **C** peuvent être réduits à l'aide d'agents réducteurs appropriés dont des exemples ont été donnés ci-dessus.

Suivant une variante de l'invention, le peptide collagénique **A**, réticulable **B** et/ou au moins en partie réticulé **C**, constitutif du moyen revendiqué, est également porteur de greffons **G** fixés sur au moins une partie des motifs amines libres de la chaîne collagénique, par l'intermédiaire de liaisons amides, **G** étant un acyle comprenant une entité hydrocarbonée, A L'EXCLUSION des restes mercaptoaminés, notamment de ceux tels que définis supra, contenant éventuellement des hétéroatomes (avantageusement O et/ou N), de préférence choisie parmi les alkyles et/ou les alcényles et/ou les alicyliques et/ou les aromatiques et, plus préférentiellement encore, parmi les groupements comprenant une chaîne alkyle, éventuellement insaturée et comprenant de 1 à 22 atomes de carbone ou répondant à la formule (**III**) suivante : 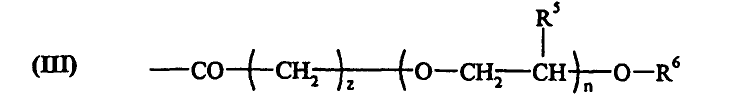 avec :
- R⁵ = H ou CH₃;
- R⁶ = H, un radical alkyle linéaire ou ramifié et de préférence un méthyle ;
- z = 0,1 ou 2 et n > 0.

Cette fonctionnalisation supplémentaire sur les sites amines des lysines peut conférer aux peptides collagéniques modifiés une aptitude supplémentaire à la réticulation ou bien encore un caractère caractère hydrophile ou hydrophobe, voire tensioactif. Il est également concevable que cette fonctionnalisation ait des visées thérapeutiques grâce à l'ancrage d'un principe actif, qui pourrait être par exemple dans l'application concernée, un ou plusieurs agents chimiques de traitement de l'anti-adhésion.

Selon une caractéristique intéressante de l'invention, ce greffon G, fixé sur les amines libres de la chaîne collagénique du peptide collagénique réticulé, modifie le caractère hydrophile/hydrophobe du produit, ce qui permet de moduler les propriétés de gonflement, de résistance mécanique et de cinétique de dégradation.

En ce qui concerne l'obtention d'un précurseur collagénique **A** porteur de restes mercaptoaminés (**I**), elle consiste essentiellement à faire réagir en solution le peptide collagénique avec au moins un précurseur d'un reste mercaptoaminé dont la fonction thiol et l'éventuelle fonction carboxylique sont bloquées, en présence d'au moins un agent de greffage, de préférence choisi dans le groupe des produits activant les groupements carboxyliques, de préférence parmi les carbodiimides. Les conditions d'obtention sont choisies de telle sorte que le greffage du reste mercaptoaminé s'opère sur les motifs acide carboxylique libres des restes acides aspartique et glutamique de la chaîne collagénique.
A cette fin, le précurseur à greffer doit posséder une fonction amine libre apte à réagir avec les COOH collagéniques pour former une liaison amide. Ce précurseur est, par exemple, une cystéine, une homocystéine ou une cystéamine, dont la fonction thiol et l'éventuelle fonction acide carboxylique est (sont) correctement protégée(s). Un moyen efficace de protection de la fonction thiol est de choisir comme résidu cystéique à greffer, la cystine, l'homocystine ou la cystamine qui comprennent toutes trois un pont disulfure stabilisateur de la fonction mercapto. Comme autre moyen de protection de cette dernière on peut choisir toute fonction classique de protection des thiols connue dans l'art antérieur (voir par exemple "Greene : *Protecting Groups in Organic Chemistry*, WILEY, 1975").

Les fonctions COOH peuvent quant à elles être protégées à l'aide d'un groupement protecteur ou toute autre fonction organique pouvant apporter une propriété quelconque intéressante (les PEG, groupements hydrophobes ou hydrophiles ou chargés).

Selon une disposition avantageuse de l'invention, le précurseur du reste mercaptoaminé à greffer répond à une formule **(IV)** correspondant à la formule **(I)** donnée supra et dans laquelle la valence libre est remplacée par un substituant apte à réagir avec les fonctions carboxyliques des acides aspartique et glutamique de la chaîne collagénique, ce substituant étant de préférence l'hydrogène, de façon à ce que la fonction réactive soit une amine primaire. Les précurseurs de formule **(IV)** tout spécialement préférés sont la cystamine **(I.1),** la cystine-diméthylester **(I.2)** et la cystine diéthylester **(I.3)** qui comprennent toutes trois un pont disulfure protégeant la fonction mercapto.

En pratique, le greffage du reste mercaptoaminé s'effectue en procédant à la dissolution du peptide collagénique puis du précurseur du reste mercaptoaminé à greffer dans un solvant approprié. Il peut s'agir, par exemple, de l'eau (de préférence) ou d'un solvant organique, comme le DiMéthylSulfOxyde (DMSO), la N-MéthylPyrrolidone (NMP) ou autres. Le précurseur du reste mercaptoaminé à greffer est toujours présent en grand excès, afin d'éviter que le collagène activé ne réagisse avec les amines contenues dans son propre squelette.
La solution réactionnelle est ensuite additionnée d'un agent de couplage, tel qu'un carbodiimide, et on laisse le greffage s'opérer en maintenant le milieu sous agitation pendant quelques heures, à température ambiante.

Ces peptides collagéniques **A** substitués par des restes mercaptoaminés précurseurs des restes thiols réticulables, sont de nouveaux produits intermédiaires stables et solubles dans l'eau. Ils peuvent être isolés et purifiés, par exemple par dialyse, diafiltration puis lyophilisation ou par précipitation en milieu organique puis par séchage.

Les précurseurs collagéniques intermédiaires **B** de type thiol, dans lesquels R² = H peuvent être obtenus par un procédé consistant essentiellement à déprotéger (transformation en thiols) les fonctions mercapto des restes mercaptoaminés greffés sur les peptides collagéniques **A** modifiés comme indiqué ci-dessus.

Dans le cas où la protection ou le masquage des fonctions mercapto est assuré par un pont disulfure (c'est-à-dire quand les précurseurs des greffons sont e.g. la cystamine ou la cystine), la régénération de la fonction thiol se fait par réduction. Cette dernière peut être réalisée à l'aide d'agents réducteurs tels que des mercaptans (mercaptoéthanol, acide mercaptoacétique, mercaptoéthylamine, benzylmercaptan, thiolcrésol, dithiothréitol, ....) et/ou des sels réducteurs (NaBH₄, Na₂SO₃.....) et/ou des réducteurs organiques (phosphine).
Avantageusement, on procède à une réduction du pont disulfure de protection en milieu aqueux basique à l'aide de dithiothréitol. Après cette étape, le collagène thiolé obtenu est purifié par dialyse/diafiltration et peut être isolé e.g. par lyophilisation.

Comme cela ressort déjà de ce qui précède, les peptides collagéniques réticulés **C** sont préparés par oxydation des fonctions thiol du peptide collagénique modifié réticulable **B**, de façon à former des ponts disulfure intercaténaires.

S'agissant de la fonctionnalisation des susdits peptides collagéniques, par des greffons G de nature différente de celle des greffons de formule **(I)** (éventuellement hydrogénés) fixés au COOH des acides aspartique et glutamique, elle consiste essentiellement :
- à procéder à une acylation d'au moins une partie des fonctions amines libres de la chaîne collagénique, de façon à fixer sur celles-ci des greffons G comprenant une entité hydrocarbonée, A L'EXCLUSION des restes mercaptoaminés, notamment ceux tels que définis supra, cette entité contenant éventuellement des hétéroatomes (avantageusement O et/ou N), de préférence choisie parmi les alkyles et/ou les alcényles et/ou les alicycliques et/ou les aromatiques et, plus préférentiellement encore, parmi les groupements comprenant une chaîne alkyle, éventuellement insaturée ou répondant à la formule **(III)** suivante : 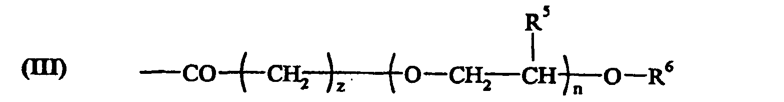 avec :
   ◇ R⁵ = H ou CH₃ ;
   ◇ R⁶ = H, un radical alkyle linéaire ou ramifié et de préférence un méthyle ;
   ◇ z = 0,1 ou 2 et n > 0 ;
- à faire réagir en solution le peptide collagénique avec au moins un précurseur d'un reste mercaptoaminé dont la fonction thiol et l'éventuelle fonction carboxylique sont bloquées, en présence d'au moins un agent de greffage, de préférence choisi dans le groupe comprenant les composés capables d'activer les groupements carboxyliques, de préférence les carbodiimides.

Pour pouvoir réagir par acylation avec les fonctions amine libres des lysines de la chaîne collagénique, les précurseurs des greffons G doivent comporter au moins une fonction acide carboxylique activable.

Les réactions d'acylation et de couplage de fonctions amine avec des sites carboxyliques appartenant à des protéines, sont connues de l'homme du métier dans le domaine de la biochimie des protéines. Pour plus de détails à cet égard, on se référera notamment aux ouvrages suivants :
- *"Techniques in protein chemistry"* R*.* L LUNDBLAD Chap. 10-14,
- *"Chemistry of protein conjugation and cross-linking*" S. S. WONG, Boca raton, CRC Press, 1993, Chap. 2.

Après cette description du produit constitutif essentiel du moyen selon l'invention, on donne dans ce qui suit des précisions quant aux caractères physiques, à la méthode de préparation et au mode d'utilisation dudit moyen pour la prévention des adhérences post-chirurgicales.

Conformément à un premier mode de réalisation du moyen selon l'invention, celui-ci se présente sous forme de film.
Par le terme "film", on entend, au sens de la présente invention, un matériau sous forme de feuille dont la surface est sensiblement plus grande que l'épaisseur.

Suivant un deuxième mode de réalisation du moyen selon l'invention, celui-ci se présente sous forme de matière fibreuse, tissée ou non-tissée, de préférence tissée et, plus préférentiellement encore, tissée avec des mailles tricotées.
A titre d'exemples de moyens selon l'invention, constitués par des matériaux collagéniques fibreux, on peut citer les nappes tissées et/ou tricotées et/ou tressées, ou bien les feutres et les mats.

Suivant un troisième mode de réalisation du moyen selon l'invention, celui-ci peut se présenter sous forme de film (ou de nappe) en matériau composite non homogène, dans lequel un renfort en polymère biodégradable, de nature collagénique ou non, est inclus dans la matrice constituée par le peptide collagénique susdéfini. Le polymère constituant le renfort peut être le peptide collagénique sélectionné conformément à l'invention, du collagène non modifié ou d'autres polymères bio-compatibles et bio-dégradables tels que des polymères d'acides α-hydroxycarboxyliques (e.g. acide polylactique et/ou polyglycolique), des celluloses ou des amidons, modifiés ou non. Il n'est pas à exclure que ces polymères d'inclusion soient des polymères synthétiques ou naturels non bio-dégradables mais bio-compatibles (e.g. silicones, ....).
Le matériau de renfort peut se présenter sous différentes formes physiques, e. g. : charges particulaires ou fibreuses, mats, feutres, matériaux tissés, tricotés, tressés,... En pratique, le moyen selon l'invention peut être, par exemple, constitué par un film composite comprenant une matrice en peptide collagénique se présentant au moins partiellement sous une forme réticulée C, telle que définie supra et un matériau de renfort constitué par un tissu ou un mat comprenant des fibres de polymères d'acides α-hydroxycarboxyliques (acide polylactique et/ou acide polyglycolique).
Ce matériau de renfort fibreux présente, e. g., la même forme que la matrice (par exemple rectangulaire), tout en ayant des dimensions légèrement plus réduites.
Selon une forme de réalisation particulière, le moyen selon l'invention peut se présenter sous forme d'un film comprenant un renfort fibreux sur une partie seulement de sa surface. En pratique, le matériau de renfort fibreux peut être évidé dans sa région centrale.
En tout état de cause, il est avantageux que le renfort fibreux soit présent sur au moins une partie des bordures du film composite, de façon à offrir une zone propre à recevoir des fils de suture.

En partant du peptide collagénique réticulé sous forme de film (éventuellement composite), il est possible de fabriquer, conformément à l'invention, des membranes suturables efficaces dans la prévention des adhérences post-chirurgicales.

De manière préférée, le moyen selon l'invention est un film composite dont la matrice est en peptide collagénique réticulé et dont le matériau de renfort se présente sous forme de matière fibreuse, tissée ou non tissée, de préférence tissée et, plus préférentiellement encore, tissé avec des mailles tricotées, ce matériau de renfort étant, en outre, avantageusement choisi parmi les (co)polymères d'acides α-hydroxycarboxyliques, de préférence les polylactiques et/ou glycoliques.

Mais il est tout à fait concevable conformément à l'invention, que le moyen de prévention des adhérences post-chirurgicales soit disposé (appliqué) et/ou implanté sur le site où il est destiné à faire fonction de barrière, non pas sous une forme solide mais sous une forme liquide (fluide ou visqueuse). D'où il s'ensuit que, selon un quatrième mode de réalisation du moyen de l'invention, celui-ci se présente sous forme non solide, réticulable et/ou au moins en partie réticulé, applicable et/ou implantable sur et/ou dans un support.

Dans ce cas, la forme non solide est, par exemple, une solution de peptide collagénique réticulable (précurseur **B**). Elle est appliquée directement sur un support (par exemple des tissus biologiques) puis est soumise à une réticulation permettant son durcissement en gel. La réticulation peut être obtenue *in situ* par action d'un agent chimique oxydant médicalement accepté, par l'oxygène de l'air ou par tout autre moyen d'oxydation facilement utilisable au cours d'une opération chirurgicale (UV, électrocoagulation...).

Dans ce quatrième mode de réalisation, la forme non-solide du peptide collagénique, comprise dans le moyen selon l'invention, peut se présenter, avantageusement, sous forme d'un liquide susceptible d'être gélifié, notamment par oxydation et/ou physiquement sous forme de gel destiné à être appliqué ainsi sur un support (e. g. tissus biologiques) où il exercera son action d'anti-adhérence.
Dans cette forme non solide gélifiée, le peptide collagénique peut, éventuellement mais non nécessairement, se trouver partiellement sous forme réticulée. Mais il va de soi que, dans cette hypothèse, les proportions de peptide collagénique réticulé C par rapport à son précurseur non réticulé **B** sont choisies de telle sorte que l'état solide n'est pas atteint.

La forme non-solide (i. e. liquide) du précurseur du peptide collagénique, à savoir e. g. une solution collagénique liquide, peut être appliquée directement à l'aide d'un outil, de préférence une seringue ou un pulvérisateur (spray). C'est ainsi que, suivant une variante avantageuse du quatrième mode de réalisation du moyen selon l'invention, ce dernier comprend au moins un outil, de préférence une seringue ou un pulvérisateur, de stockage et d'application dans et/ou sur un support, de la forme non solide du peptide collagénique réticulable et/ou au moins en partie réticulé, telle que définie ci-dessus.

Dans le cas où le moyen selon l'invention est un pulvérisateur de peptide collagénique modifié sous forme liquide (spray), la pulvérisation peut être assurée par un système pompe/buse et/ou par un agent propulseur gazeux.

Le support peut s'entendre ici comme étant des tissus biologiques (par exemple animaux), que l'on souhaite panser et/ou que l'on traite, e. g. *in vivo,* en vue de prévenir les adhérences post-chirurgicales.

Eventuellement, les propriétés rhéologiques de la solution collagénique peuvent être ajustées par ajout d'un polymère naturel ou synthétique biocompatible, de préférence biodégradable (comme par exemple les polysaccharides, les glycosaminoglycanes, les protéines ou glycoprotéines, les polymères éthoxylés tel le polyéthylène glycol...)

Lorsqu'un agent chimique est utilisé pour réticuler la solution, il est choisi, de façon avantageuse, parmi les oxydants employés de façon usuelle en médecine comme par exemple l'eau oxygénée.

A ce quatrième mode de réalisation du moyen de l'invention (liquide/gel de peptide collagénique), peut être associé un procédé de réalisation d'un moyen de prévention des adhérences post-chirurgicales sur des tissus vivants, caractérisé en ce qu'il consiste, essentiellement :
- à mettre en oeuvre un fluide collagénique, e. g. une solution, du peptide collagénique du type de celui défini ci-dessus ;
- à appliquer ce fluide sur des tissus biologiques, par exemple à l'aide d'une seringue ou d'un pulvérisateur ;
- et à faire en sorte qu'intervienne une réticulation du fluide collagénique *in situ*, par exemple à l'aide d'un oxydant biocompatible (e. g. oxygène de l'air, H₂O₂ ...).

Selon une première manière de mise en oeuvre de ce procédé, le mélange entre l'oxydant et le fluide, par exemple la solution collagénique, peut être réalisée avant le dépôt. Dans ce cas, la solution appliquée est déjà partiellement réticulée au moment du dépôt. L'homogénéité du mélange solution/oxydant peut être assurée par un dispositif d'application tel qu'un système de double seringue équipée d'une tête de mélange (dispositif similaire à celui utilisé pour les colles biologiques à base de fibrine), un double pulvérisateur équipé d'une buse de mélange ou tout autre dispositif permettant d'assurer un mélange homogène.

Selon une deuxième manière de mise en oeuvre de ce procédé, l'application est réalisée en deux temps. De préférence, le dépôt du fluide, par exemple de la solution collagénique liquide, est effectué en premier et on procède ensuite au dépôt de la solution oxydante. Mais l'ordre inverse d'addition peut également être utilisé.

Suivant un cinquième mode de réalisation du moyen selon l'invention, ce dernier se présente sous forme d'un bloc homogène ou composite, non assimilable à un film.

Selon un autre de ses aspects, la présente invention concerne un procédé de préparation des moyens pour la prévention des adhérences post-chirurgicales tels que décrits ci-dessus.

Conformément à une première voie de mise en oeuvre correspondant à la préparation des formes solides (par exemple films ou blocs homogènes ou composites : 1^{er}, 3^{ème} et 5^{ème} modes de réalisation), le procédé comprend les étapes essentielles suivantes :
1. réaliser une solution, de préférence aqueuse, de précurseur réticulable de peptide collagénique modifié ;
2. éventuellement filtrer cette solution de façon à en extraire les éléments de taille supérieure ou égale à 0,8µm de préférence à 0,45µm et plus préférentiellement encore à 0,2 µm ;
3. mettre en forme le filtrat dans la configuration visée pour le moyen de prévention des adhérences post-chirurgicales à préparer ;
4. éventuellement gélifier la solution mise en forme, lors d'une phase de maturation, par abaissement de sa température en dessous de sa température de gélification ;
5. éventuellement éliminer le solvant, de préférence par évaporation ;
6. faire en sorte qu'intervienne la réticulation, de préférence par oxydation ;
7. le cas échéant, éliminer par lavages successifs l'éventuel agent oxydant employé ;
8. éventuellement imprégner le matériau réticulé ou en cours de réticulation à l'aide d'une solution d'au moins un agent plastifiant (par exemple : glycérol, polyéthylène glycol de basse masse moléculaire) ;
9. éventuellement sécher le matériau réticulé ;
10. éventuellement découper le matériau aux dimensions de l'application ;
11. éventuellement stériliser par rayonnement le matériau réticulé.

L'un des intérêts du procédé de préparation selon l'invention tient à ce qu'il peut être mis en oeuvre de manière simple, stérile et économique.

L'étape 1 de mise en solution s'effectue par exemple à l'aide d'un solvant constitué par de l'eau stérile, à une température comprise entre 20 et 50 °C et un pH compris entre 6 et 8. Suivant une caractéristique préférée de cette première voie du procédé selon l'invention, la solution réalisée à l'étape 1 a un titre en précurseur réticulable de peptide collagénique supérieur ou égal à 1 % et, de préférence, compris entre 1 et 15 %.

L'étape 2, facultative mais néanmoins préférée de filtration, est un moyen d'épuration voire de stérilisation de la solution préparée à l'étape 1. Grâce à la qualité de solubilité dans l'eau du précurseur du peptide collagénique réticulé, sélectionné selon l'invention, il est possible de filtrer les solutions de précurseur sur des filtres dont la porosité est inférieure à 0,8 microns, de préférence ≤ 0,45 microns et, plus préférentiellement encore, de l'ordre de 0,2 microns. Cette faculté de pouvoir stériliser par filtration est un atout important sur le plan industriel.

L'étape 3 consiste à mettre en forme la solution filtrée de façon à obtenir un objet réticulé de forme et de dimension contrôlées. Pour la mise en oeuvre de l'étape 3, on peut, par exemple, couler la solution filtrée dans des moules, de préférences stériles. Selon une variante de cette étape, on peut aussi déposer la solution filtrée sur un support plan stérile par enduction. Selon une autre variante, on peut assurer la mise en forme, par extrusion de la solution à travers une filière de forme appropriée au moyen visé, par exemple rectangulaire. Dans ce dernier cas, une certaine solidification doit intervenir lors de l'extrusion pour que celle-ci soit possible.

La maturation facultative, mais néanmoins préférée, de l'étape 4 (ou gélification physique) de la solution, consiste à laisser reposer pendant plusieurs heures, voire plusieurs jours, la solution mise en forme à une température inférieure à sa température de gélification ou température de transition gel/solide, qui peut être, par exemple, comprise entre 20 et 30 °C, dans le cas d'une solution aqueuse de peptide collagénique.

L'élimination du solvant, de préférence de l'eau, selon l'étape 5 est une phase facultative, qui peut être effectuée pendant ou après la gélification, de préférence après. Il peut s'agir, de préférence, d'une évaporation à température donnée en présence éventuelle d'agents dessicants.

La réticulation selon l'étape 6 est réalisée sur des articles mis en forme, éventuellement gélifiés, se présentant avantageusement sous forme sèche. La réticulation consiste à oxyder le précurseur de type thiol du peptide collagénique à l'aide d'un oxydant tel que l'iode ou H₂O₂. Après plusieurs minutes de contact avec l'oxydant, il est préférable de réaliser (étape 7) des lavages successifs des articles ainsi façonnés. Ceux-ci peuvent ensuite être transformés à souhait en différents moyens pour la prévention des adhérences post-chirugicales.

A l'issue de ces étapes, les matériaux réticulés obtenus sont dans un état hydraté. Ils peuvent éventuellement être imprégnés (étape 8) à l'aide d'un matériau plastifiant couramment utilisé pour la mise en forme des matériaux collagéniques (glycérol, polyéthylène glycol de basse masse moléculaire). Le matériau plastifiant peut également être introduit dès l'étape 1 de préparation de la solution.

Enfin, les matériaux collagéniques chargés ou non en plastifiant(s) peuvent être séchés (étape 9).

Bien que ce procédé puisse être réalisé à l'aide de moyens stériles et comporter éventuellement une étape de filtration stérilisante, on peut prévoir une étape supplémentaire de stérilisation (étape 10), par exemple aux rayonnements β.

Les articles obtenus, par exemple les films, sont stables à l'état sec sur de longues durées et sont manipulables après réhydratation dans tout liquide aqueux approprié.

Selon une variante de cette première voie de préparation du moyen de l'invention, on peut, par exemple, inclure un renfort au cours de l'étape 3, soit en coulant la solution filtrée de collagène sur le renfort déposé dans le moule ou sur le support, soit en déposant le renfort sur la solution déjà coulée et éventuellement gélifiée. Dans ce dernier cas une deuxième couche de solution collagénique peut être à nouveau déposée. Selon le mode d'introduction du renfort, on peut ainsi obtenir une membrane présentant une face "peptide collagénique" et une face "renfort" ou une membrane tricouche "peptide collagénique/renfort/peptide collagénique". Ces exemples ne sont pas limitatifs et l'on peut envisager d'autres variantes du procédé permettant d'obtenir des matériaux composites à partir du peptide collagénique susdéfini.

Conformément à une deuxième voie de mise en oeuvre, correspondant aux formes non-solides (liquides) des moyens selon l'invention, le procédé comprend les étapes essentielles suivantes :
1. réaliser une solution, de préférence aqueuse, de précurseur réticulable de peptide collagénique modifié ;
2. éventuellement filtrer cette solution de façon à en extraire les éléments de taille supérieure ou égale à 0,8µm de préférence à 0,45µm et plus préférentiellement encore à 0,22 µm ;
3. éventuellement concentrer la solution ;
4. conditionner stérilement la solution sous atmosphère inerte.

Suivant une caractéristique préférée de cette deuxième voie du procédé selon l'invention, la solution réalisée à l'étape 1 a un titre en précurseur réticulable de peptide collagénique supérieur ou égale à 1 % et, de préférence, compris entre 1 et 15 % et un pH compris entre 4 et 10 et, de préférence, compris entre 6 et 8.
Au cours de l'*étape* 1, il est possible d'ajouter à la solution des polymères synthétiques ou naturels biocompatibles ou des petites molécules biocompatibles pouvant modifier la rhéologie de la solution et retarder la gélification du matériau (par exemple urée).
En pratique, les quantités de polymères incorporées sont telles que lesdits polymères représentent, dans la forme finale réticulée du moyen de prévention, entre 1 et 50 % de la matière sèche totale et, de préférence, entre 1 et 20 %. L'étape 1 est, de préférence, réalisée en conditions stériles, à partir de polymères (précurseurs de peptide collagénique et éventuels polymères additifs) stériles.
L'*étape* 2 de filtration est facultative et non nécessaire si l'étape 1 a été réalisée dans des conditions stériles. Dans le cas où la filtration a lieu, elle est réalisée à une température supérieure à 35 °C et de préférence comprise entre 40 et 50 °C.
L'*étape 3* de concentration est facultative. Elle est réalisée stérilement par évaporation partielle du solvant, de façon à obtenir des solutions concentrées ayant un titre en précurseur réticulable de peptide collagénique compris entre 5 et 30 % et, de préférence, entre 10 et 20 %
L'*étape 4* de conditionnement est réalisée de manière stérile sous une atmosphère inerte (azote ou argon). La solution est conditionné dans un contenant qui peut être la - forme définitive d'application (seringue ou pulvérisateur) ou un flacon de stockage.

Il est à noter qu'à température ambiante la forme liquide du moyen peut se présenter sous forme gélifiée. Ce gel, caractéristique des produits collagéniques, peut être refluidifié après agitation quelques secondes à 40 °C.

Cette solution peut, ensuite, être réticulée par oxydation en un gel chimique.

Selon une troisième voie de mise en oeuvre, qui combine les deux premières, on applique la solution conditionnée obtenue à l'issue de l'étape 4 de la deuxième voie sur un support et on fait en sorte qu'intervienne une réticulation, de préférence à l'aide d'un oxydant biocompatible.

Un autre aspect de l'invention couvre l'utilisation des moyens susdécrits (film, liquide/gel à base du peptide collagénique) pour la prévention des adhérences post-opératoires.

Les moyens de l'invention ont ceci d'avantageux que leur mise en place, au cours de l'acte chirurgical, est aisée. Elle dépend de la forme physique et chimique du moyen (membrane réticulée ou forme liquide non réticulée).

Les membranes peuvent être découpées extemporanément aux dimensions de la zone lésée à protéger. Les membranes sont appliquées entre les tissus biologiques susceptibles de donner des adhérences chirurgicales. Elles peuvent être mises en place sous forme sèches ou peuvent être hydratées, juste avant la mise en place, dans du sérum physiologique. Leur maintien peut être assuré par des sutures ou par application préalable d'une colle chirurgicale (à base de fibrine ou de matériaux collagéniques).
Selon une variante d'utilisation, la membrane peut être roulée sur elle même et introduite à l'aide d'un trocart, selon une technique classique de chirurgie sous coelioscopie.

S'agissant des moyens non solides (liquides/gels) de prévention des adhérences post-chirurgicales, ils peuvent être appliqués, notamment, de deux manières qui sont définies ci-dessous..
Selon une première manière d'application, la forme liquide peut être déposée directement sur les tissus lésés à l'aide d'une seringue, d'un pulvérisateur ou de tout autre dispositif d'application. Selon la composition de la formulation et le mode d'application, il peut être nécessaire de réchauffer la solution à 40 °C pendant quelques secondes avant l'application. La réticulation est alors assurée *in situ* par application dans un deuxième temps d'un agent chimique oxydant acceptable en chirurgie, tel qu'une solution diluée d'eau oxygénée. Selon des variantes de ce mode d'application, l'oxygène de l'air, les rayonnements UV ou d'autres sources d'énergie pouvant conduire à une oxydation, peuvent être utilisés.
Selon une deuxième manière d'application, la forme liquide (précurseur) du peptide collagénique non réticulé est mélangée juste avant l'application, avec un agent chimique oxydant, à l'aide d'un dispositif approprié (double seringue équipée d'une tête de mélange, double pulvérisateur avec buse de mélange ou autre dispositif de mélange).
Ces deux manières d'applications peuvent être également adaptées pour être utilisées en chirurgie coelioscopique.
De manière plus générale, l'invention concerne un procédé de prévention des adhérences post-chirurgical est caractérisé en ce que l'on applique (injection ou pulvérisation e. g.) la solution conditionnée sur un support (tissus biologiques) et en ce que l'on fait en sorte qu'intervienne une réticulation, de préférence grâce à un oxydant biocompatible.

Il ressort de la description qui précède que les moyens pour la prévention des adhérences post-chirurgicales selon l'invention sont, par exemple :
- soit des moyens solides, tels que des films ou des membranes éventuellement composites et/ou multicouches, comprenant essentiellement le peptide collagénique au moins partiellement réticulé tel que défini supra,
- soit des moyens non-solides (liquides/gels) comprenant le même matériau collagénique sous forme non réticulée et destiné à être appliqué et à réticuler *in vivo,* de façon à former *in situ*, sur le site d'action (tissus biologiques), les moyens solides de prévention des adhérences post-chirurgicales.
Grâce à la nature de leur matériau constitutif essentiel, ces moyens sont dotés, sous forme réticulée, d'excellentes propriétés de résistance mécanique, de barrière physique contre l'adhésion, de bio-compatibilité, de bio-résorbabilité, de non toxicité et de mise en oeuvre de préparations industrielles, ces propriétés étant tout à fait supérieures à celle des moyens selon l'art antérieur.

Les exemples qui suivent permettront de bien comprendre l'invention dans tous ses aspects et de faire ressortir tous ses avantages et ses variantes de mise en oeuvre.

### EXEMPLES

### PARTIE I : SYNTHESE DES MOYENS

### EXEMPLE 1 : SYNTHESE D'UN PEPTIDE COLLAGENIQUE (FORME B) DONT LES ACIDES CARBOXYLIQUES SONT SUBSTITUES PAR LA CYSTEINE-ETHYLESTER (TAUX DE SUBSTITUTION REPRESENTANT 7 % MOLAIRE DES ACIDES AMINES).

### 1) Etape I : couplage (obtention forme A):

25 g d'atélocollagène (types I + III, extrait de peaux de veaux, 1,3 mmol de COOH/g) sont placés dans 2,5 l d'eau et la température du milieu est amené à 50 °C sous agitation. La solution à 1 % p/v ainsi obtenue est filtrée sur 0,22 µm.
Une fois la température abaissée à 30 °C, 46,5 g de cystine-diéthylester sont ajoutés et le pH est ajusté à 4,2. On ajoute alors 12 g de 1-(3-diméthylaminopropyl)-3-éthylcarbodiimide HCl et on laisse la réaction se dérouler pendant 2 h à 30 °C sous agitation. Le milieu réactionnel est concentré à 5 % p/v et dialysé contre de l'eau pour éliminer l'excès de réactifs et les sous-produits de la réaction.
Le produit obtenu est un intermédiaire stable de synthèse. Il s'agit d'un peptide collagénique (forme **A**) dont une fraction des acides aspartiques et glutamiques sont substitués par de la cystine-diéthylester. Il peut être isolé par lyophilisation ou être réduit pour conduire au collagène thiol correspondant (forme **B**).

### 2) Etape II : réduction (obtention forme B) :

Au peptide collagénique modifié en solution à 5 % p/v dans l'eau obtenu à l'étape I, on ajoute 7,6 g de glycine, 5,8 g de 1,4-dithiothréitol et qsp NaOH 4 N pour atteindre un pH de 9,0. Le milieu réactionnel est maintenu trois heures sous agitation à 35 °C. A ce stade, la solution est acidifiée à pH 2 par HCl 6 N, dialysée contre HCl 0,012 N pour éliminer toute trace de réactifs et de sous-produits de réaction, puis filtrée sur 0,22 µm. Le produit ainsi purifié est isolé par lyophilisation.
Le taux de substitution est mesuré par un dosage par l'acide 5,5'-dithiobis-2-nitrobenzoïque (DTNB), réactif spécifique des fonctions thiol. Ce dosage est décrit dans : "Ellman G. L., Tissue sulfhydryl groups, *Archives of Biochemistry and Biophysics*, 1959, 82, 70-77".
[SH] : 0,706 mmol/g de produit sec, soit 7 % molaire d'acides aminés substitués.
Toute la synthèse peut être réalisée aseptiquement de manière à obtenir *in fine* le produit sous forme d'un lyophilisat stérile.

### EXEMPLE 2 : PREPARATION D'UN FILM DE COLLAGENE MODIFIE, RETICULE A L'IODE, POUR LA PREVENTION DES ADHERENCES POSTOPERATOIRES.

### Etape 1 :

Une solution à 20g/l de peptide collagénique précurseur selon l'exemple 1, est préparée par dissolution du lyophilisat dans de l'eau stérile. Dans cet exemple 2,0 g de lyophilisat sont dissous dans 98 g d'eau stérile. La solution est agitée dans un récipient fermé à 40 °C pendant 15 min, afin d'obtenir une dissolution complète. Le pH de la solution est ajusté à 6,5 avec de la soude 1 N, à 25 °C. La solution est remise en agitation à 40 °C pendant 10 min.

### Etape 2 :

La solution est filtrée à 40 °C sur membranes de porosité 0,45 µm, puis sur membranes de porosité 0,2 µm. La dernière filtration s'effectue au dessus des moules stériles (on peut utiliser des boîtes de Petri en polystyrène).

### Etape 3 :

40,0 g de solution filtrée sont coulés dans deux moules de 12 × 12 cm². Les moules sont refermés.

### Etape 4 :

On réalise la maturation de la solution, qui se traduit par une gélification physique, pendant 24 h à une température de 16°C ± 1. Cette température est nécessairement inférieure à la température de transition gel/sol. La maturation est effectuée dans une enceinte à température régulée, les moules reposent sur une plaque horizontale.

### Etape 5 :

Après 24 h, les couvercles des moules sont retirés et l'évaporation des solutions gélifiées s'effectue sur 24 h, à la même température dans une enceinte confinée, en présence d'agents dessicants (typiquement des pastilles de soude). Au bout de 24 h, les films obtenus sont secs, limpides et lisses.

### Etape 6 :

La réticulation des films secs est effectuée à 20 °C, en versant 30 g de solution iodoalcoolique, obtenue par dissolution de 1,0 g d'iode dans 100 ml d'éthanol et 30 g d'eau est nécessaire pour obtenir un film réticulé.
Le film réticulé est retiré de la solution iodée.

### Etape 7 :

On effectue des lavages successifs par des solutions d'éthanol à 80 %, puis au tampon phosphate, jusqu'à décoloration totale du film.
Toutes les solutions utilisées sont stériles.

### Etape 8 :

Dans cet exemple, aucune étape d'imprégnation par un agent plastifiant n'est réalisée.

### Etape 9 :

Le film est ensuite laissé à sécher sous hotte à flux laminaire pendant 24 h. Le film séché obtenu contient un pourcentage d'eau résiduelle de 10 % environ.

### Etape 10 :

Le film est ensuite découpé facilement aux dimensions adéquates pour l'application.

### Etape 11 :

Chaque pièce est conditionnée individuellement dans un sachet pour être stérilisée par rayonnements β.
Les films obtenus sont stables à température ambiante pendant plusieurs mois. Ils restent stables et manipulables après 24 h dans l'eau ou dans un tampon phosphate.

### EXEMPLE 3 : PREPARATION D'UN FILM DE COLLAGENE MODIFIE, RETICULE AU PEROXYDE D'HYDROGENE, POUR LA PREVENTION DES ADHERENCES POSTOPERATOIRES.

Le procédé est identique quelque soit la nature de la matière première (collagène) et le taux de greffage du peptide collagénique précurseur. On reprend ici les étapes 1 à 5 de l'exemple 3 jusqu'à l'obtention des films secs non réticulés.

### Etape 6' :

La réticulation des films secs est effectuée à 20 °C, en versant 30 g de solution de peroxyde d'hydrogène à 0,3 % dans une solution aqueuse décimolaire d'acétate d'ammonium.

### Etape 7 :

Le film réticulé est retiré et lavé successivement par 30g de tampon phosphate pH 7,4 et 30 g d'eau.
Toutes les solutions utilisées sont stériles.

### Etape 8 :

Dans cet exemple, aucune étape d'imprégnation par un agent plastifiant n'est réalisée.

### Etape 9 :

Le film est ensuite laissé à sécher sous hotte à flux laminaire pendant 24 h. Le film séché obtenu contient un pourcentage d'eau résiduelle de10 % environ.

### Etape 10 :

Le film est ensuite découpé facilement aux dimensions adéquates pour l'application.

### Etape 11 :

Chaque pièce est conditionnée individuellement dans un sachet pour être stérilisée aux rayonnements béta.
Les films obtenus sont stables à température ambiante. Ils restent stables et manipulables après 24 h dans l'eau ou dans un tampon phosphate.

### EXEMPLE 4 : PROPRIETES MECANIQUES EN TRACTION DES FILMS OBTENUS SELON L'EXEMPLE 2.

Les mesures de propriétés mécaniques de films de collagène réticulé sont effectuées à l'aide d'un appareil d'essais universel de type DY34 de la marque Adamel Lhomargy. Les films sont hydratés à température ambiante dans un tampon phosphate salin (PBS, pH = 7,4) pendant 2 h. Puis ils sont découpés en bandes de 4 mm sur 30 mm à l'aide d'un emporte-pièce très coupant. L'épaisseur est mesurée sur les échantillons hydratés. Les échantillons sont fixés sur un cadre carton qui aide à la mise en place dans les mâchoires. L'échantillon de film est maintenu hydraté. Le cadre est coupé juste avant l'essai de traction, qui se déroule à vitesse constante de 2 mm/min.
Le module à l'origine ainsi que la contrainte à la rupture sont calculés d'après les courbes de traction en utilisant les sections des éprouvettes hydratées.
Les propriétés en traction des films obtenus selon le procédé décrit à l'exemple 3 dépendent de la matière première (collagène ou gélatine) et du taux de greffage des peptides collagéniques précurseurs.
Le tableau suivant décrit les propriétés d'un film obtenu à partir de l'exemple 2.

| EPAISSEUR SECHE (µM) | EPAISSEUR HUMIDE (µM) | **Fmax** (N) | ALLONGEMENT (%) | σ max (MPA) | MODULE INITIAL (MPA) |
|---|---|---|---|---|---|
| 45 | 80 | 5,4 . | 42,5 | 16,7 | 25,8 |
| Légende : Fmax = force maximale à la rupture σ max = contrainte maximale à la rupture | | | | | |

### EXEMPLE 5 : PREPARATION D'UN FILM COMPOSITE RETICULE COLLAGENE MODIFIE / TREILLIS SYNTHETIQUE, POUR LA PREVENTION DES ADHERENCES POST-OPERATOIRES.

Le procédé de préparation du film est identique quelque soit la nature de la matière première (collagène) et le taux de greffage du peptide collagénique précurseur. Les étapes 1 et 2 sont identiques à celles décrites à l'exemple 3.
L'étape 3 est modifiée de la façon suivante.
Un treillis synthétique (par exemple treillis en polyester biodégradable disponible dans le commerce, de dimension 10 x 10 cm² et d'épaisseur 200 µm environ) est disposé dans chacun des moules (12 × 12 cm²).
40,0 g de solution filtrée sont coulés sur le treillis. Les moules sont refermés.
Les étapes 4 et 5 sont identiques à celles décrites à l'exemple 3.
L'oxydation peut être réalisée par une solution iodée ou le peroxyde d'hydrogène. Les étapes 6 à 11 sont selon la méthode choisie identiques à celles de l'exemple 3 ou de l'exemple 4.
Les films obtenus sont stables à température ambiante. Ils restent stables et manipulables après 24 h dans l'eau ou dans un tampon phosphate. Ils présentent des propriétés mécaniques supérieures à celle des membranes non renforcées. Ils présentent de plus une très bonne résistance au déchirement qui permet une grande aisance de suturabilité. Ils peuvent être enroulés sur eux même ce qui permet de les introduire dans des trocarts.

### EXEMPLE 6 : PREPARATION D'UNE FORME LIQUIDE A PARTIR DE COLLAGENE MODIFIE, RETICULABLE IN SITU, POUR LA PREVENTION DES ADHERENCES POST-OPERATOIRES.

Le procédé de préparation de la forme liquide est identique quelle que soit la nature de la matière première (collagène) et le taux de greffage du peptide collagénique précurseur.
Pour cet exemple, le peptide collagénique précurseur (exemple 1) se présente sous forme d'un lyophilisat stérile.

### Etape 1 :

Une solution de collagène modifié à 85 g/l est préparée par dissolution en conditions stérile du lyophilisat (exemple 1) dans de l'eau stérile. Dans cet exemple, 1,50 g de lyophilisat à 11 % de taux d'humidité sont dissous dans 8,3 g d'eau stérile (préalablement dégazée par courant stérile d'azote). La solution est agitée dans un récipient fermé à 45 °C pendant 15 min, afin d'obtenir une dissolution complète. Le pH de la solution est ajusté à 7 avec 5,9 ml de soude 0,1 N stérilisés par filtration sur 0,2 microns.

### Etapes 2 et 3 :

Les étapes de filtration et concentration n'ont pas lieu pour cet exemple, étant donné que le lyophilisat de départ est stérile et mis en solution à la concentration finale visée dans des conditions stériles.

### Etape 4 :

La solution est répartie en seringues stériles de 1 ml, refermées hermétiquement.

### EXEMPLE 7 :

On reproduit à la différence près que, dans l'étape 4, la solution est conditionnée stérilement, non pas en seringues, mais dans un flacon pulvérisateur.

### PARTIE II : EVALUATION BIOLOGIQUE

### EXEMPLE 8 : EXPERIMENTATION D'UN FILM DE COLLAGENE SELON L'INVENTION, TEL QU'OBTENU DANS L'EXEMPLE 3.

Cette étude a pour objectif d'évaluer les propriétés de prévention des adhérences post-chirurgicales d'un film de collagène selon l'invention, tels qu'obtenus dans l'exemple 3.
Cette étude est réalisée chez la rate, en site péritonéal, pour un délai d'observation de 2 semaines minimum.
L'efficacité du produit est estimée par observation macroscopique des éventuelles adhérences sur les lieux d'application. Seront étudiées : la fréquence, l'étendue et la sévérité des adhérences développées.
Des lésions traumatiques de la séreuse péritonéale sont pratiquées au niveau des cornes utérines et d'une fenêtre pariétale chez la rate.
Le film est positionné de façon à séparer les lésions pariétales et utérines engendrées pour un délai minimum de 2 semaines. Sur chaque rate, sont réalisés un site témoin et un site test.
Passées les 2 semaines minimum, les animaux sont sacrifiés par injection d'une dose létale de barbituriques.
La sévérité des adhérences est évaluée par cotation de 0 à 3 :
- 0 : absence d'adhérence,
- 1 : adhérences discrètes, clivables par dissection mousse,
- 2 : adhérences modérées, nécessitant une dissection tranchante partielle,
- 3 : adhérences sévères, non clivables sans dissection tranchante étendue.

Pour exprimer les résultats, les cotations des 10 sites témoins sont additionnées puis la moyenne est déterminée. Cette valeur moyenne correspond donc à la sévérité moyenne des adhésions.
Pour les témoins la cotation moyenne obtenue est de 2,2. De plus, tous les sites étudiés présentent des adhésions sur 95 % de la surface de la lésion.
Pour les sites tests la cotation moyenne est de 0,22.
On notera que seulement 3 sites sur 10 sites étudiés présentaient des adhésions, et que ces adhésions n'étaient présentes que sur environ 10 % de la surface de la lésion.
En conclusion, on obtient une diminution de 90 % de la force des adhésions. Ainsi qu'une diminution très importante du nombre et de la surface de ces adhésions.

### EXEMPLE 9 : EXPERIMENTATION D'UN FILM DE COLLAGENE MODIFIE / TREILLIS SELON L'INVENTION, TEL QU'OBTENU DANS L'EXEMPLE 5.

Cette étude a pour objectif d'évaluer les propriétés de prévention des adhérences post-chirurgicales de film de collagène modifié / treillis selon l'invention décrite dans l'exemple 5.
Cette étude est réalisée chez la rate, en site péritonéal, pour un délai d'observation de 2 semaines minimum.
L'efficacité du produit est estimée par observation macroscopique des éventuelles adhérences sur les lieux d'application. Seront étudiées : la fréquence, l'étendue et la sévérité des adhérences développées.
Des lésions traumatiques de la séreuse péritonéale sont pratiquées au niveau des cornes utérines et d'une fenêtre pariétale chez la rate. Le film de collagène modifié /treillis est alors positionné de façon à séparer les lésions pariétales et utérines engendrées pour un délai minimum de 2 semaines. Sur chaque rate, sont réalisés un site témoin et un site test.
Passées les 2 semaines minimum, les animaux sont sacrifiés par injection d'une dose létale de barbituriques.
La sévérité des adhérences est évaluée par cotation de 0 à 3 :
- 0 : absence d'adhérence,
- 1 : adhérences discrètes, clivables par dissection mousse,
- 2 : adhérences modérées, nécessitant une dissection tranchante partielle,
- 3 : adhérences sévères, non clivables sans dissection tranchante étendue.

Pour exprimer les résultats, les cotations des 10 sites témoins sont additionnées puis la moyenne est déterminée. Cette valeur moyenne correspond donc à la sévérité moyenne des adhésions.
Pour les témoins la cotation moyenne obtenue est de 2,2. De plus tous les sites étudiés présentent des adhésions sur 0 % de la surface de la lésion.
Pour les sites tests la cotation moyenne est de 0,1.
On notera que seulement 3 sites sur 20 sites étudiés présentaient des adhésions, et que ces adhésions n'étaient présentes que sur environ 10 % de la surface de la lésion.
En conclusion, on obtient une diminution de 96 % de la force des adhésions. Ainsi qu'une diminution très importante de la surface des adhésions présentes.

### EXEMPLE 10 : EXPERIMENTATION DE LA FORME LIQUIDE SELON L'INVENTION, TELLE QU'OBTENUE DANS L'EXEMPLE 6

Cette étude a pour objectif d'évaluer les propriétés de prévention des adhérences post-chirurgicales de la forme liquide selon l'invention décrite dans l'exemple 6.
Cette étude est réalisée chez la rate, en site péritonéal, pour un délai d'observation de 2 semaines minimum.
L'efficacité du produit est estimée par observation macroscopique des éventuelles adhérences sur les lieux d'application. Seront étudiées : la fréquence, l'étendue et la sévérité des adhérences développées.
Des lésions traumatiques de la séreuse péritonéale sont pratiquées au niveau des cornes utérines et d'une fenêtre pariétale chez la rate. Avant l'utilisation de la solution, les seringues sont réchauffées au bain-marie à 37 °C, afin de recouvrer une solution visqueuse. Cette solution peut être appliquée sur un support. La viscosité de cette solution lui permet de former un dépôt, tout en garantissant une épaisseur suffisante. 300 µl de cette solution est alors positionné de façon à séparer les lésions pariétales et utérines engendrées pour un délai minimum de 2 semaines. La réticulation du dépôt est effectuée par vaporisation d'une solution à 1 % de peroxyde d'hydrogène. La gélification du dépôt est presque instantanée. On obtient un hydrogel de collagène homogène. Sur chaque rate, sont réalisés un site témoin et un site test.
Passées les 2 semaines minimum, les animaux sont sacrifiés par injection d'une dose létale de barbituriques.
La sévérité des adhérences est évaluée par cotation de 0 à 3 :
- 0 : absence d'adhérence,
- 1 : adhérences discrètes, clivables par dissection mousse,
- 2 : adhérences modérées, nécessitant une dissection tranchante partielle,
- 3 : adhérences sévères, non clivables sans dissection tranchante étendue.

Pour exprimer les résultats, les cotations des 10 sites témoins sont additionnées puis la moyenne est déterminée. Cette valeur moyenne correspond donc à la sévérité moyenne des adhésions.
Pour les témoins la cotation moyenne obtenue est de 2,2. De plus tout les sites étudiés présentent des adhésions sur 90 % de la surface de la lésion.
Pour les sites tests (300 µl d'hydrogel de collagène) la cotation moyenne est de 0,7. On notera que seulement 7 sites sur 10 sites étudiés présentaient des adhésions, et que ces adhésions n'étaient présentes que sur environ 30 % de la surface de la lésion.
En conclusion, on obtient une diminution de 70 % de la force des adhésions. Ainsi qu'une diminution très importante de la surface des adhésions présentes.

## Revendications

1. Moyen pour la prévention des adhérences post-chirurgicales, **caractérisé en ce qu'**il comprend au moins un peptide collagénique modifié par greffage de fonctions thiol libres ou substituées, réticulable et/ou au moins en partie réticulé et dont les fonctions thiol sont apportées par des restes mercaptoaminés exclusivement greffés sur les acides aspartique et glutamique des chaînes collagéniques, par l'intermédiaire de liaisons amides.

2. Moyen selon la revendication 1 **caractérisé en ce qu'**au moins une partie du peptide collagénique modifié est sous forme de précurseur A sur lequel sont greffés des restes mercaptoaminés porteurs de fonctions thiols substituées, au moins une partie de ces restes mercaptoaminés répondant à la formule générale (I) suivante : 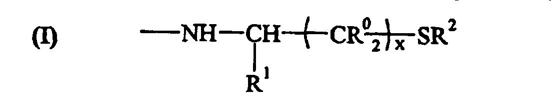 dans laquelle :
• x = 1 ou 2,
• R⁰ = H ou CH₃,
• R¹ représente H ou COOR³ avec R³ correspondant à un radical hydrocarboné de type aliphatique, aromatique ou alicyclique, de préférence alkylique, alcénylique, arylique, aralkylique, alkylarylique, aralcénylique, alcénylarylique; et plus préférentiellement encore de type méthylique ou éthylique;
• R² est un radical aliphatique et/ou alicylique et/ou aromatique, de préférence un alkyle ou un acyle éventuellement soufré et/ou aminé, et plus préférentiellement encore R² répond à la formule (**II**) suivante : 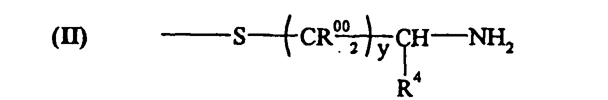
• avec y, R⁰⁰ et R⁴ répondant à la même définition que celle donnée en légende dans la formule (I) pour x, R⁰ et R¹.

3. Moyen selon la revendication 2, **caractérisé en ce que** les restes mercaptoaminés greffés sur la forme A du peptide collagénique sont choisis dans le groupe des radicaux suivants :

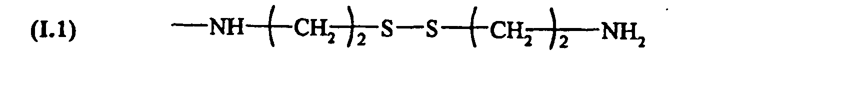

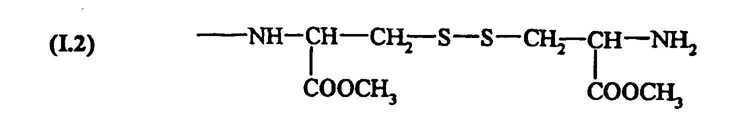

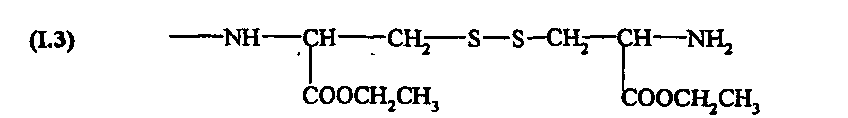

4. Moyen selon la revendication 1, **caractérisé en ce qu'**au moins une partie du peptide collagénique est sous une forme **B** de précurseur réticulable intermédiaire de type thiol sur lequel sont greffés des restes mercaptoaminés dont au moins une partie répondent à la formule générale **(I)** donnée dans la revendication 2 et dans laquelle R² = H et dans laquelle R³ peut représenter, en outre, l'hydrogène ou un cation apte à former un sel avec COO⁻, ce cation étant, de préférence, Na⁺, K⁺, Li⁺.

5. Moyen selon la revendication 1, **caractérisé en ce qu'**au moins une partie du peptide collagénique modifié est une forme **C** réticulée comprenant des chaînes collagéniques reliées entre elles par des ponts disulfure dont les atomes de soufre constitutifs appartiennent à des restes mercaptoaminés exclusivement greffés, sur les acides aspartique et glutamique des chaînes collagéniques, par l'intermédiaire de liaisons amides.

6. Moyen selon la revendication 5, **caractérisé en ce que** le peptide collagénique sous forme **C** est obtenu à partir du peptide collagénique **B** selon la revendication 4.

7. Moyen selon l'une quelconque des revendications 1 à 4, **caractérisé en ce qu'**au moins une partie du peptide collagénique **(A** et/ou **B** et/ou **C)** est également porteur de greffons **G** fixés sur au moins une partie des motifs amines libres de la chaîne collagénique, par l'intermédiaire de liaisons amides, **G** étant un acyle comprenant une entité hydrocarbonée, A L'EXCLUSION des restes mercaptoaminés, notamment de ceux tels que définis supra, cette entité contenant éventuellement des hétéroatomes (avantageusement O et/ou N) et étant, de préférence, choisie parmi les alkyles et/ou les alcényles et/ou les alicyliques et/ou les aromatiques et, plus préférentiellement encore, parmi les groupements comprenant une chaîne alkyle, éventuellement insaturée, ou répondant à la formule **(III)** suivante : 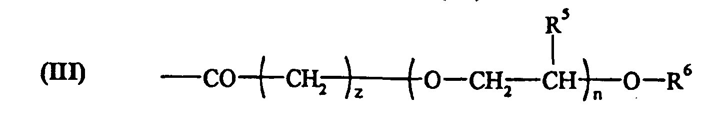 avec :
• R⁵ = H ou CH₃ ;
• R⁶ = H, un radical alkyle linéaire ou ramifié et de préférence un méthyle ;
• z = 0,1 ou 2 et n > 0.

8. Moyen selon l'une quelconque des revendications 1 à 7, **caractérisé en ce qu'**il se présente sous forme de film.

9. Moyen selon l'une quelconque des revendications 1 à 8, **caractérisé en ce qu'**il comprend un composite comportant, d'une part, une matrice comprenant le peptide collagénique tel que défini dans les revendications 1 à 7 et, d'autre part, un matériau de renfort inclus dans cette matrice, ce renfort étant choisi parmi les polymères biodégradables.

10. Moyen selon l'une quelconque des revendications 1 à 9, **caractérisé en ce que** le renfort se présente sous forme de matière fibreuse, tissée ou non-tissée, de préférence tissée et, plus préférentiellement encore, tissée avec des mailles tricotées.

11. Moyen selon la revendication 9 ou la revendication 10, **caractérisé en ce que** le matériau de renfort est choisi parmi les (co)polymères d'acides α-hydroxycarboxyliques, de préférence les polylactiques et/ou glycoliques.

12. **-** Moyen selon la revendication 9, **caractérisé en ce qu'**il se présente sous forme d'un film comprenant un renfort fibreux sur une partie seulement de sa surface.

13. Moyen selon l'une quelconque des revendications 1 à 7, **caractérisé en ce qu'**il se présente sous forme non solide, réticulable et/ou au moins en partie réticulé, applicable et/ou implantable sur et/ou dans un support.

14. Moyen selon la revendication 13, **caractérisé en ce qu'**il comprend du peptide collagénique sous forme liquide.

15. **-** Moyen selon la revendication 13, **caractérisé en ce qu'**il comprend du peptide collagénique sous forme de gel.

16. **-** Moyen selon l'une quelconque des revendications 13 à 15, **caractérisé en ce qu'**il comprend au moins un outil - de préférence une seringue ou un pulvérisateur - de stockage et d'application dans et/ou sur un support, d'une forme non solide (telle que définie dans la revendication 13) du peptide collagénique réticulable et/ou au moins en partie réticulé.

17. **-** Moyen selon la revendication 16, **caractérisé en ce qu'**il comprend un agent d'oxydation permettant la réticulation du peptide collagénique.

18. **-** Procédé de préparation du moyen pour la prévention des adhérences post-chirurgicales selon l'une quelconque des revendications 1 à 12, **caractérisé en ce qu'**il comprend les étapes essentielles suivantes :
**1.** réaliser une solution, de préférence aqueuse, de précurseur réticulable de peptide collagénique modifié ;
**2.** éventuellement filtrer cette solution de façon à en extraire les éléments de taille supérieure ou égale à 0,8µm de préférence à 0,45µm et plus préférentiellement encore à 0,2 µm ;
**3.** mettre en forme le filtrat dans la configuration visée pour le moyen de prévention des adhérences post-chirurgicales à préparer ;
**4.** éventuellement gélifier la solution mise en forme, lors d'une phase de maturation, par abaissement de sa température en dessous de sa température de gélification ;
**5.** éventuellement éliminer le solvant, de préférence par évaporation ;
**6.** faire en sorte qu'intervienne la réticulation, de préférence par oxydation ;
**7.** le cas échéant, éliminer par lavages successifs l'éventuel agent oxydant employé ;
**8.** éventuellement imprégner le matériau réticulé ou en cours de réticulation à l'aide d'une solution d'au moins un agent plastifiant (par exemple : glycérol, polyéthylène glycol de basse masse moléculaire) ;
**9.** éventuellement sécher le matériau réticulé ;
**10.** éventuellement découper le matériau aux dimensions de l'application ;
**11.** éventuellement stériliser par rayonnement le matériau réticulé.

19. **-** Procédé de préparation du moyen pour la prévention des adhérences post-chirurgicales selon la revendication 13, **caractérisé en ce qu'**il comprend les étapes essentielles suivantes :
**1.** réaliser une solution, de préférence aqueuse, de précurseur réticulable de peptide collagénique modifié ;
**2.** éventuellement filtrer cette solution de façon à en extraire les éléments de taille supérieure ou égale à 0,8µm de préférence à 0,45µm et plus préférentiellement encore à 0,22 µm ;
**3.** éventuellement concentrer la solution ;
**4.** conditionner stérilement la solution sous atmosphère inerte.

20. **-** Procédé selon la revendication 18 ou 19, **caractérisé en ce que** la solution réalisée à l'étape 1 a un titre en précurseur réticulable de peptide collagénique modifié :
• supérieur ou égal à 1 %,
• de préférence compris entre 1 et 15 %.

21. Procédé selon la revendication 19 ou 20, **caractérisé en ce que** l'on applique la solution conditionnée sur un support et **en ce que** l'on fait en sorte qu'intervienne une réticulation, de préférence grâce à un oxydant biocompatible.

## Patentansprüche

1. Mittel zur Verhinderung von post-chirurgischen Verklebungen,
**dadurch gekennzeichnet,**
**dass** es wenigstens ein Kollagen-Peptid umfasst, das durch Pfropfen von freien oder substituierten Thiol-Funktionen modifiziert ist, das vernetzbar und/oder wenigstens teilweise vernetzt ist, und wobei die Thiolfunktionen durch Merkaptoamin-Reste bereitgestellt sind, die ausschließlich auf die Asparaginsäuren und Glutaminsäuren der Kollagen-Ketten durch Amid-Bindungen gepfropft sind.

2. Mittel nach Anspruch 1,
**dadurch gekennzeichnet,**
**dass** wenigstens ein Teil des modifizierten Kollagen-Peptids in Form des Vorläufers A vorliegt, auf den Merkaptoamin-Reste gepfropft sind, welche substituierte Thiol-Funktionen tragen, wobei wenigstens ein Teil dieser Merkaptoamin-Reste die folgende allgemeine Formel **(I)** aufweist: 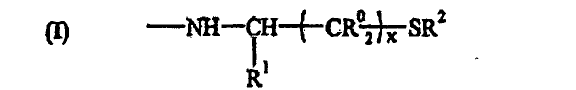 worin
• x = 1 oder 2,
• R⁰ = H oder CH₃,
• R¹ H oder COOR³ bedeutet, wobei R³ einen aliphatischen, aromatischen oder alicyklischen Kohlenwasserstoff-Rest, bevorzugt Alkyl, Alkenyl, Aryl, Aralkyl, Alkylaryl, Aralkenyl, Alkenylaryl und noch weiter bevorzugt Methyl oder Ethyl bedeutet,
• R² ein aliphatischer und/oder alicyklischer und/oder aromatischer Rest ist, bevorzugt ein Alkyl- oder ein Acyl-Rest, der gegebenenfalls Schwefel- und/oder Amino-Gruppen enthält und noch weiter bevorzugt weist R² die folgende Formel (II) auf: 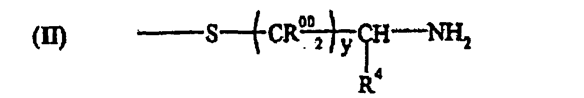
• worin für y, R⁰⁰ und R⁴ dieselbe Definition gilt wie für x, R⁰ und R¹ bezüglich Formel (I) angegeben.

3. Mittel nach Anspruch 2,
**dadurch gekennzeichnet,**
**dass** die auf die Form A des Kollagen-Peptids gepfropften Merkaptoamin-Reste ausgewählt sind aus der Gruppe der folgenden Reste:

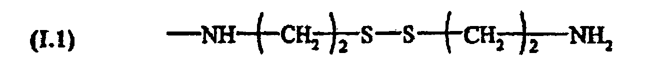

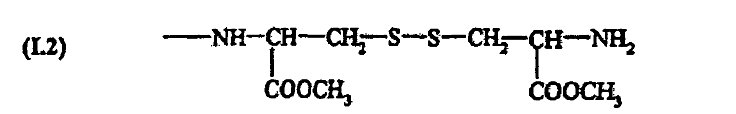

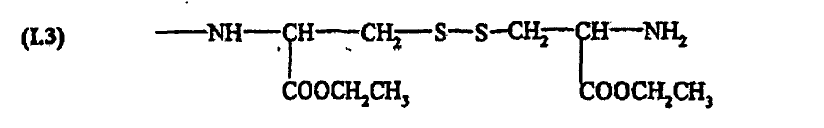

4. Mittel nach Anspruch 1,
**dadurch gekennzeichnet,**
**dass** wenigstens ein Teil des Kollagen-Peptids in einer Form **B** eines intermediären vernetzbaren Vorläufers vom Typ Thiol vorliegt, auf welchen Merkaptoamin-Reste gepfropft sind, von denen wenigstens ein Teil die in Anspruch 2 angegebene allgemeine Formel (**I**) aufweist und worin R² = H und worin R³ unter anderem Wasserstoff oder ein Kation bedeuten kann, das in der Lage ist mit COO⁻ ein Salz zu bilden, wobei dieses Kation bevorzugt Na⁺, K⁺, Li⁺ ist.

5. Mittel nach Anspruch 1,
**dadurch gekennzeichnet,**
**dass** wenigstens ein Teil des modifizierten Kollagen-Peptids in einer vernetzten Form **C** vorliegt, welche Kollagen-Ketten umfasst, die miteinander durch Disulfid-Brücken verbunden sind, wobei die Schwefelatome, welche die Brücken bilden, zu Merkaptoamin-Resten gehören, die ausschließlich auf die Asparaginsäuren und Glutaminsäuren der Kollagen-Ketten über Amid-Bindungen gepfropft sind.

6. Mittel nach Anspruch 5,
**dadurch gekennzeichnet,**
**dass** das Kollagen-Peptid der Form **C** aus dem Kollagen-Peptid **B** nach Anspruch 4 erhalten ist.

7. Mittel nach einem der Ansprüche 1 bis 4,
**dadurch gekennzeichnet,**
**dass** wenigstens ein Teil des Kollagen-Peptids (**A** und/oder **B** und/oder **C**) ebenfalls Pfropf-Gruppen G trägt, die an wenigstens einem Teil der freien Amin-Gruppen der Kollagen-Kette über Amid-Bindungen befestigt sind, wobei G ein Acyl-Rest ist, der eine Kohlenwasserstoff-Einheit umfasst,
wobei Merkaptoamin-Reste, insbesondere solche wie oben definiert, ausgeschlossen sind, wobei diese Einheit gegebenenfalls Heteroatome (vorteilhafterweise O und/oder N) enthält, und bevorzugt ausgewählt ist aus Alkyl und/oder Alkenyl und/oder alicyklischen und/oder aromatischen Resten und noch weiter bevorzugt aus Gruppierungen die eine gegebenenfalls ungesättigte Alkylkette umfassen oder die folgende Formel (III) aufweisen: 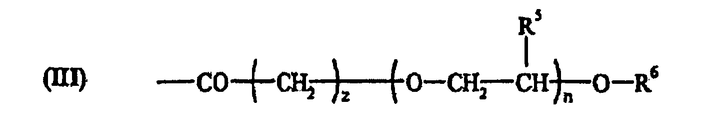 worin
• R⁵ = H oder CH₃;
• R⁶ = H, ein linearer oder verzweigter Alkylrest und bevorzugt Methyl ist;
• z = 0, 1 oder 2 und n > 0.

8. Mittel nach einem der Ansprüche 1 bis 7,
**dadurch gekennzeichnet,**
**dass** es in Form eines Films vorliegt.

9. Mittel nach einem der Ansprüche 1 bis 8,
**dadurch gekennzeichnet,**
**dass** es einen Verbundstoff umfasst, der einerseits eine Matrix, die das wie in einem der Ansprüche 1 bis 7 definierte Kollagen-Peptid umfasst und andererseits ein in dieser Matrix enthaltenes Verstärkungsmaterial umfasst, wobei dieses Verstärkungsmaterial aus biologisch abbaubaren Polymeren ausgewählt ist.

10. Mittel nach einem der Ansprüche 1 bis 9,
**dadurch gekennzeichnet,**
**dass** das Mittel in Form eines fasrigen, gewebten oder nicht gewebten Materials, vorzugsweise in gewebter Form und noch weiter bevorzugt in gewebter Form mit gestrickten Maschen vorliegt.

11. Mittel nach Anspruch 9 oder Anspruch 10,
**dadurch gekennzeichnet,**
**dass** das Verstärkungsmaterial ausgewählt ist aus (Co)Polymeren von α-Hydroxy-carbonsäuren, vorzugsweise Polymilchsäuren und/oder Polyglycolsäuren.

12. Mittel nach Anspruch 9,
**dadurch gekennzeichnet,**
**dass** es in Form eines Films vorliegt, der nur auf einer Seite seiner Oberfläche ein fasriges Verstärkungsmaterial aufweist.

13. Mittel nach einem der Ansprüche 1 bis 7,
**dadurch gekennzeichnet,**
**dass** es in nicht fester, vernetzbarer und/oder wenigstens teilweise vernetzter Form vorliegt und auf und/oder in einem Träger anwendbar und/oder implantierbar ist.

14. Mittel nach Anspruch 13,
**dadurch gekennzeichnet,**
**dass** es ein Kollagen-Peptid in flüssiger Form umfasst.

15. Mittel nach Anspruch 13,
**dadurch gekennzeichnet,**
**dass** es ein Kollagen-Peptid in Gelform umfasst.

16. Mittel nach einem der Ansprüche 13 bis 15,
**dadurch gekennzeichnet,**
**dass** es wenigstens ein Werkzeug umfasst - vorzugsweise eine Spritze oder einen Zerstäuber - zur Lagerung und Anwendung in und/oder auf einem Träger, in Form eines nicht festen (wie in Anspruch 13 definiert) vemetzbaren und/oder wenigstens teilweise vernetzten Kollagen-Peptids.

17. Mittel nach Anspruch 16,
**dadurch gekennzeichnet,**
**dass** es ein Oxidationsmittel umfasst, das die Vernetzung des Kollagen-Peptids ermöglicht.

18. Verfahren zur Herstellung eines Mittels zur Verhinderung post-chirurgischer Verklebungen nach einem der Ansprüche 1 bis 12,
**dadurch gekennzeichnet,**
**dass** es die folgenden wesentlichen Schritte umfasst:
1. Bereitstellen einer bevorzugt wässrigen Lösung von vemetzbarem Vorläufer eines modifizierten Kollagen-Peptids;
2. gegebenenfalls Filtrieren dieser Lösung um Bestandteile mit einer Größe von 0,8 µm oder größer, bevorzugt größer oder gleich 0,45 µm und noch weiter bevorzugt größer oder gleich 0,2 µm zu entfernen;
3. Formen des Filtrats in die für das herzustellende Mittel zur Verhinderung von post-chirurgischen Verklebungen vorgesehene Konfiguration;
4. gegebenenfalls Gelieren der geformten Lösung bei einem Sättigungsstadium, indem die Temperatur der Lösung unter ihre Geliertemperatur emiedrigt wird;
5. gegebenenfalls Entfernen des Lösungsmittels, vorzugsweise durch Verdampfen;
6. Bewirken der Vernetzung, vorzugsweise durch Öxidation;
7. gegebenenfalls Entfernen des verwendeten optionalen Oxidationsmittes durch aufeinanderfolgendes Spülen;
8. gegebenenfalls Imprägnieren des Materials in vernetztem Zustand oder im Verlauf der Vernetzung mit Hilfe einer Lösung wenigstens eines Weichmachers (beispielsweise: Glycerin, Polyethylenglycol mit geringer Molekülmasse);
9. gegebenenfalls Trocknen des vernetzten Materials;
10. gegebenenfalls Zuschneiden des Materials zu Abmessungen für die Anwendung;
11. gegebenenfalls Sterilisieren des vernetzten Materials durch Bestrahlen.

19. Verfahren zur Herstellung eines Mittels zur Verhinderung von post-chirurgischen Verklebungen nach Anspruch 13,
**dadurch gekennzeichnet,**
**dass** es die folgenden wesentlichen Schritte umfasst:
1. Bereitstellen einer bevorzugt wässrigen Lösung von vemetzbarem Vorläufer eines modifizierten Kollagen-Peptids;
2. gegebenenfalls Filtrieren dieser Lösung um Bestandteile mit einer Größe von 0,8 µm oder größer, bevorzugt größer oder gleich 0,45 µm und noch weiter bevorzugt größer oder gleich 0,2 µm zu entfernen;
3. gegebenenfalls Konzentrieren der Lösung;
4. steriles Konditionieren der Lösung in inerter Atmosphäre.

20. Verfahren nach Anspruch 18 oder 19, **dadurch gekennzeichnet, dass** die in Schritt 1 gebildete Lösung einen Gehalt an vernetzbarem Vorläufer eines modifizierten Kollagen-Peptids aufweist, der:
• größer oder gleich 1 % ist,
• vorzugsweise zwischen 1 und 15 % liegt.

21. Verfahren nach Anspruch 19 oder 20,
**dadurch gekennzeichnet,**
**dass** man die konditionierte Lösung auf einen Träger aufträgt und eine Vernetzung bewirkt, vorzugsweise durch ein biokompatibles Oxidationsmittel.

## Claims

1. Means for preventing post-surgical adhesions, **characterized in that** it comprises at least one collagenic peptide which is modified by grafting free or substituted thiol functions, which is crosslinkable and/or at least partly crosslinked and the thiol functions of which are provided by mercaptoamino residues exclusively grafted onto the aspartic and glutamic acids of the collagenic chains, via amide bonds.

2. Means according to Claim 1, **characterized in that** at least some of the modified collagenic peptide is in the form of a precursor **A** onto which are grafted mercaptoamino residues bearing substituted thiol functions, at least some of these mercaptoamino residues corresponding to the following general formula (**I**) : 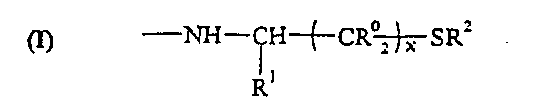 in which:
• x = 1 or 2,
• R⁰ = H or CH₃,
• R¹ represents H or COOR³ with R³ corresponding to a hydrocarbon-based radical of the aliphatic, aromatic or alicyclic type, preferably of the alkyl, alkenyl, aryl, aralkyl, alkylaryl, aralkenyl or alkenylaryl type, and even more preferably of the methyl or ethyl type;
• R² is an aliphatic and/or alicyclic and/or aromatic radical, preferably an alkyl or an acyl, optionally a sulphur-containing and/or amino- alkyl or acyl, and even more preferably R² corresponds to the following formula (**II**): 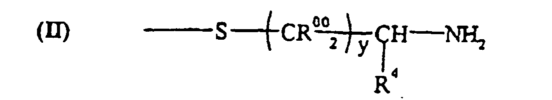
• with y, R⁰⁰ and R⁴ corresponding to the same definition as that given in the legend in formula (**I**) for x, R⁰ and R¹.

3. Means according to Claim 2, **characterized in that** the mercaptoamino residues grafted onto the **A** form of the collagenic peptide are chosen from the group of the following radicals:

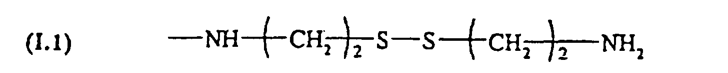

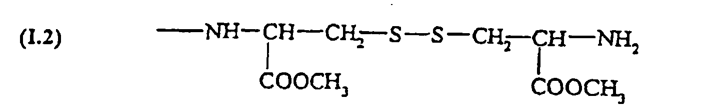

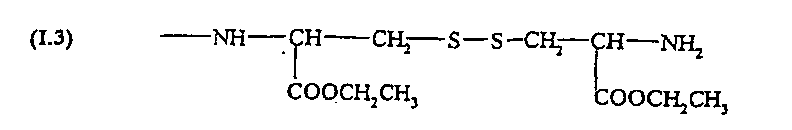

4. Means according to Claim 1, **characterized in that** at least some of the collagenic peptide is in a thiol-type intermediate crosslinkable precursor form **B,** onto which are grafted mercaptoamino residues, at least some of which correspond to the general formula (I) given in Claim 2 and in which R² = H and in which R³ may also represent hydrogen or a cation capable of forming a salt with COO⁻, this cation preferably being Na⁺, K⁺, Li⁺.

5. Means according to Claim 1, **characterized in that** at least some of the modified collagenic peptide is in a crosslinked form **C** comprising collagenic chains attached to one another by disulphide bridges, the constituent sulphur atoms of which belong to mercaptoamino residues exclusively grafted onto the aspartic and glutamic acids of the collagenic chains, via amide bonds.

6. Means according to Claim 5, **characterized in that** the collagenic peptide in **C** form is obtained from the collagenic peptide **B** according to Claim 4.

7. Means according to any one of Claims 1 to 4, **characterized in that** at least some of the collagenic peptide (**A** and/or **B** and/or **C**) also carries grafts G attached to at least some of the free amine units of the collagenic chain, via amide bonds, G being an acyl comprising a hydrocarbon-based entity, EXCLUDING mercaptoamino residues, in particular those as defined above, this entity optionally containing hetero atoms (advantageously O and/or N), and preferably being chosen from alkyls and/or alkenyls and/or alicyclics and/or aromatics, and even more preferably from the groups comprising an alkyl chain, optionally unsaturated or corresponding to the following formula (**III**): 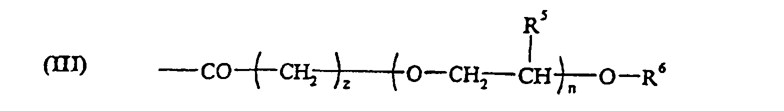 with:
• R⁵ = H or CH₃;
• R⁶ = H, or a linear or branched alkyl radical, and preferably a methyl;
• z = 0, 1 or 2 and n > 0;

8. Means according to any one of Claims 1 to 7, **characterized in that** it is in the form of a film.

9. Means according to any one of Claims 1 to 8, **characterized in that** it comprises a composite comprising, firstly, a matrix comprising the collagenic peptide as defined in Claims 1 to 7 and, secondly, a reinforcement material included in this matrix, this reinforcement being chosen from biodegradable polymers.

10. Means according to any one of Claims 1 to 9, **characterized in that** the reinforcement is in the form of a fibrous substance, which is woven or nonwoven, preferably woven, and even more preferably woven with knitted stitches.

11. Means according to Claim 9 or Claim 10, **characterized in that** the reinforcement material is chosen from α-hydroxycarboxylic acid (co)polymers, preferably poly(lactic acid)s and/or poly(glycolic acid)s.

12. Means according to Claim 9, **characterized in that** it is in the form of a film comprising a fibrous reinforcement on only part of its surface.

13. Means according to any one of Claims 1 to 7, **characterized in that** it is in a nonsolid form which is crosslinkable and/or at least partly crosslinked and which can be applied and/or implanted onto and/or into a support.

14. Means according to Claim 13, **characterized in that** it comprises collagenic peptide in liquid form.

15. Means according to Claim 13, **characterized in that** it comprises collagenic peptide in the form of a gel.

16. Means according to any one of Claims 13 to 15, **characterized in that** it comprises at least one tool - preferably a syringe or a spray - for storing and for applying into and/or onto a support, a nonsolid form (as defined in Claim 13) of the crosslinkable and/or at least partly crosslinked collagenic peptide.

17. Means according to Claim 16, **characterized in that** it comprises an oxidizing agent for crosslinking the collagenic peptide.

18. Process for preparing the means for preventing post-surgical adhesions according to any one of Claims 1 to 12, **characterized in that** it comprises the following essential steps:
1. preparing a solution, preferably an aqueous solution, of crosslinkable precursor of modified collagenic peptide;
2. optionally filtering this solution so as to extract therefrom the elements which are greater than or equal to 0.8 µm, preferably greater than or equal to 0.45 µm, and even more preferably greater than or equal to 0.2 µm in size;
3. moulding the filtrate in the intended configuration for the means for preventing post-surgical adhesions to be prepared;
4. optionally gelling the moulded solution, in a maturation phase, by decreasing its temperature below its gelling temperature;
5. optionally eliminating the solvent, preferably by evaporation;
6. bring about the crosslinking, preferably by oxidation;
7. where appropriate, eliminating, with successive washes, the oxidizing agent possibly used;
8. optionally impregnating the material which is crosslinked or which is in the process of being crosslinked, using a solution of at least one plasticizer (for example: glycerol, low molecular weight polyethylene glycol);
9. optionally drying the crosslinked material;
10. optionally cutting the material to the size for use;
11. optionally sterilizing the crosslinked material by radiation.

19. Process for preparing the means for preventing post-surgical adhesions according to Claim 13, **characterized in that** it comprises the following essential steps:
1.preparing a solution, preferably an aqueous solution, of crosslinkable precursor of modified collagenic peptide;
2. optionally filtering this solution so as to extract therefrom the elements which are greater than or equal to 0.8 µm, preferably greater than or equal to 0.45 µm, and even more preferably greater than or equal to 0.22 µm in size;
3. optionally concentrating the solution;
4. packaging the solution sterilely under an inert atmosphere.

20. Process according to Claim 18 or 19, **characterized in that** the solution prepared in step 1 has a titre in terms of crosslinkable precursor of the collagenic peptide:
• greater than or equal to 1%,
• preferably between 1 and 15%.

21. Process according to Claim 19 or 20, **characterized in that** the packaged solution is applied onto a support and **in that** crosslinking is brought about, preferably using a biocompatible oxidizing agent.
